# DEMANDE DE BREVET EUROPEEN

(11) **EP 1 371 351 A1**
(43) Date de publication de la demande: **17.12.2003**
(21) Numéro de dépôt: 03291347.7
(22) Date de dépôt: 05.06.2003
(51) Int. Cl.: A61K 7/02

(54) **Utilisation de polymères du méthacrylate de méthyle comme agent de texture dans des compositions cosmétiques ou pharmaceutiques et compositions les contenant**

(30) Priorité: 12.06.2002 FR 0207198
(71) Demandeur: SOCIETE D'EXPLOITATION DE PRODUITS POUR LES INDUSTRIES CHIMIQUES, S.E.P.P.I.C., 75321 Paris Cédex 07 (FR)
(72) Inventeur: Roso, Alicia, 81710 Saix (FR); Bulcourt, Catherine, 92100 Boulogne Billancourt (FR); Michel, Nelly, 94700 Maisons Alfort (FR)
(74) Mandataire: Conan, Philippe Claude

(57) **Abrégé**

Utilisation d'un polymère ou d'un mélange de polymères choisis parmi les copolymères ou les terpolymères du méthacrylate de méthyle avec un ou plusieurs de monomères choisis parmi les esters de l'acide acrylique ou de l'acide méthacrylique, comme agent de texture dans des compositions cosmétiques ou pharmaceutiques formulées sous forme de poudres applicables sur le corps humain ou animal. Composition cosmétique ou pharmaceutique en contenant et leur utilisation comme produits de maquillage.

## Description

L'invention a pour objet l'utilisation de poudres de polymères pour préparer des compositions cosmétiques plus particulièrement destinées au maquillage de la peau.

Les compositions de maquillage de la peau contiennent généralement des agents de texture constitués de grains de poudre sphériques ou lamellaires pour les rendre douces lors de l'application sur la peau. Cependant, ces poudres sont difficiles à mettre en oeuvre dans la préparation des poudres pressées, qui est une des formulations cosmétiques les plus communes de ces compositions de maquillage.

En effet, ces agents de texture sont des charges solides dites "incompactables", c'est à dire qu'elles ne permettent pas l'obtention d'un compactage satisfaisant dans les procédés classiques de compression. On entend par compactage satisfaisant, une poudre pressée présentant une surface lisse et plane et conservant avec sa compacité dans le temps tout en présentant une résistance aux chocs suffisante.

Avec de telles charges dites "incompactables", on observe des débuts de fragmentation et de morcellement de la poudre pressée, dès que la quantité mise en oeuvre dépasse 1% en poids de la poudre pressée à cause de l'apparition d'un phénomène de relaxation des particules.

Comme exemples de charges dites incompactables il y a :
les polyméthylméthacrylates, souvent désignés dans la littérature par PMMA et qui sont formés de microsphères microporeuses de surface spécifique supérieure ou égale à 0,5 m² par gramme, comme celles commercialisées sous les noms MICROPEARL^{TM} M 305 et MICROPEARL^{TM} M100 ;
les microsphères de silice comme celles commercialisées sous les noms Silica beads^{TM} ou Polytrap^{TM} ;
les microsphères creuses en matériau thermoplastique comme les polyéthylènes, les polystyrènes, les polyacrylonitriles ou les polyamides, comme celles commercialisées sous les noms Orgasol^{TM} ou encore en polyesters comme celles commercialisées sous le nom Expancel^{TM};
les microcapsules en matériau organique ou inorganique comme celles commercialisées sous le nom Macrolite^{TM} ; ou bien
certaines charges lamellaires comme le Sericite BC282^{TM}, le Talc EXTRA STEAMICOOS^{TM}.

Aujourd'hui on ne sait incorporer plus de 1 % en poids de tels agents de texture dans une formulation de maquillage, qu'en mettant en oeuvre un procédé de fabrication particulier incluant une étape d'extrusion. Un tel procédé est décrit dans la demande de brevet européen publiée sous le numéro EP 0 651 991 A1. Cette étape d'extrusion impliquant l'utilisation d'un appareillage spécifique limite donc l'utilisation de telles charges, notamment dans la petite et moyenne industrie cosmétique qui ne dispose généralement que de moyens de compression usuels.

C'est pourquoi la demanderesse s'est attachée à rechercher de nouveaux agents de textures qui n'aient pas les inconvénients présentés ci-dessus. Elle a trouvé que, contrairement à toute attente, certains copolymères de méthacrylate de méthyle, grâce à leur souplesse, leur capacité intrinsèque à se déformer et leur élasticité plus grande, pouvaient être plus efficacement comprimés, en favorisant l'étape de déformation plastique nécessaire à une bonne cohésion de leur poudre de particules sphériques et en réduisant les phénomènes de relaxation en fin de compression, lesdits phénomènes étant responsables des effets de morcellement et de fragmentation de la poudre pressée obtenue.

C'est pourquoi l'invention a pour objet l'utilisation d'un polymère ou d'un mélange de polymères choisis parmi les copolymères ou les terpolymères du méthacrylate de méthyle avec un ou plusieurs monomères choisis parmi l'acrylate de butyle, l'acrylate de (1-méthyl propyle), l'acrylate de (2-méthyl propyle), l'acrylate de (1,1-diméthyl éthyle), le méthacrylate de butyle, le méthacrylate de (1-méthyl propyle), le méthacrylate de (2-méthyl propyle) ou le méthacrylate de (1,1-diméthyl éthyle), comme agent de texture dans des compositions cosmétiques ou pharmaceutiques formulées sous forme de poudre applicables sur le corps humain ou animal.

Par poudre, on signifie dans le cadre de la présente invention qu'il peut s'agir, ou bien d'une poudre dispersée dans une phase aqueuse ou organique telle une cire, une huile, un solvant organique ou un mélange de ces phases ou dans une émulsion de type eau dans huile, huile dans eau ou multiple, voire dans un milieux anhydre solidifié comme un mascara, un vernis ongles, un fond teint fluide, un rouge à lèvres ou un stick fond de teint ou bien d'une poudre, qu'elle soit sous forme libre ou compactée, comme les poudres libres ou pressées unifiantes du teint, les talcs ou poudres corporelles, les fonds de teint en cake, les blush, les ombres à paupières.

Les polymères utilisés, comme défini précédemment, sont connus et leur préparation est par exemple décrite dans la demande de brevet européen EP 1 041 095. Ils se présentent sous forme de microsphères.

L'invention a plus particulièrement pour objet l'utilisation telle que définie précédemment, dans laquelle les compositions cosmétiques ou pharmaceutiques sont des poudres pressées.

Par poudre pressée, on entend dans le cadre de la présente invention, une tablette compactée obtenue par compression mécanique du mélange de poudres initial, ledit état compact devant se maintenir au cours du temps, ladite tablette devant avoir une surface plane et lisse, posséder une résistance suffisante au choc pour ne pas se casser, et dont la poudre constitutive peut être aisément prélevée au moyen d'un doigt, d'un applicateur mousse ou d'un pinceau, pour être appliquée sur la peau.

Selon un premier aspect particulier de la présente invention, celle-ci a pour objet l'utilisation telle que définie précédemment, d'un copolymère du méthacrylate de méthyle avec l'un ou l'autre des monomères choisis parmi l'acrylate de butyle, l'acrylate de (1-méthyl propyle), l'acrylate de (2-méthyl propyle) ou l'acrylate de (1,1-diméthyl éthyle).

Selon un mode tout particulier de la présente invention, il s'agit de l'utilisation, telle que définie précédemment, d'un copolymère de méthacrylate de méthyle et d'acrylate de butyle.

Les polymères de ce type tout particulier sont commercialisés sous le nom MICROSPHERE^{TM}.

L'invention a aussi pour objet une composition cosmétique ou pharmaceutique formulée sous forme d'une poudre, applicable sur le corps humain ou animal, caractérisée en ce qu'elle comprend :
- une quantité efficace d'un polymère ou d'un mélange de polymères tel que défini précédemment, et,
- au moins un composé cosmétiquement acceptable.

Par au moins un composé cosmétiquement acceptable, on entend dans le cadre de la présente invention, au moins un composé choisi parmi notamment, les colorants, les pigments, les liants hydrophobes comme les esters d'acides gras, les cires, les charges minérales, végétales ou de synthèse, les filtres solaires UV-A ou UV-B, les parfums, et leurs agents de fixation, des agents conservateurs ou des agents de textures.

Comme pigments, il y a plus particulièrement les pigments pouvant être mis en oeuvre dans les formulations cosmétiques, pharmaceutiques destinées à être appliquées sur la peau comme, par exemple, le dioxyde de titane, l'oxychlorure de bismuth, les oxydes de fer marrons, les oxydes de fer jaune, les oxydes de fer noir, ou les oxydes de fer rouges ou encore les pigments nacrés blancs ou colorés tels que les Mica-Titane.

Comme cires, il y a par exemple la cire d'abeilles, l'Ozokérite ,la cire de Carnauba ou de Candelilla.

Comme charge, il y a par exemple la cellulose microcrystalline, la méthyl cellulose, le kaolin, le talc, les micas, l'amidon, comme l'amidon de riz ou le carbonate de magnésium, l'hydrocarbonate de magnésium, la silice, les dérivés de polyethylène, le carbonate de calcium, les oxydes de zinc ou de titane ou les sels métalliques d'acides gras.

Comme esters d'acides gras, il y a par exemple les composés de formule (I):

R₁-(C=O)-O-[[CH₂-CH[O-[C(=O)]ₘ-R₂]-CH₂-O]ₙ-[C(=O)]ₚ]_{q}-R₃ (I)

dans laquelle :
R₁ représente une chaîne hydrocarbonée saturée ou insaturé, linéaire ou ramifiée comportant de 7 à 30 atomes de carbone,
R₂ représente indépendamment de R₁, un atome d'hydrogène, une chaîne hydrocarbonée saturée ou insaturée linéaire ou ramifiée comportant de 7 à 30 atomes de carbone,
R₃ représente indépendamment de R₁ ou de R₂, un atome d'hydrogène ou une chaîne hydrocarbonée saturée ou insaturée, linéaire ou ramifiée comportant de 1 à 30 atomes de carbone,
m, n, p et q sont indépendamment l'un de l'autre égaux à 0 ou à 1, étant entendu que lorsque R₃ représente un atome d'hydrogène, q est différent de 0.

Dans la formule (I) telle que définie précédemment, R₁, R₂ et R₃ représentent généralement, indépendamment les uns des autres, un radical choisi parmi les radicaux heptyle, octyle, nonyle, décyle, undécyle, dodécyle, tridécyle, tétradécyle, pentadécyle, hexadécyle, heptadécyle, octadécyle, nonadécyle, eicosyle, uneicosyle, docosyle, heptadécènyle, eicosènyle, uneicosènyle, docosènyle ou heptadécadiènyle ou décènyle.

Le groupe R₁-C(=O)- représente plus particulièrement l'un des radicaux octanoyle (caprylyle), décanoyle, undécylènoyle, dodécanoyle (lauroyle), tétradécanoyle (myristyle), hexadécanoyle (palmitoyle), octadécanoyle (stéaryle), eicosanoyle (arachidoyle), docosanoyle (béhènoyle), 8-octadécènoyle (oléyle), éicosènoyle (gadoloyle), 13-docosènoyle (érucyle), 9,12-octadécadiènoyle (linoléoyle) ou 9,12,15-octadécatriénoyle (linolénoyle)

Comme exemples de composés de formule (I) telle que définie ci-dessus il y a plus particulièrement les composés de formule (la) :

R₁-(C=O)-O-CH₂-CH[O-[C(=O)]ₘ-R₂]-CH₂-O-[C(=O)]ₚ-R₃ (Ia)

correspondant à la formule (I) telle que définie précédemment dans laquelle q et n sont égaux à 1, ou un mélange de composés de formules (la) et de préférence,
ou bien d'un composé de formule (la₁) :

R₁-(C=O)-O-CH₂-CH(OH)-CH₂-OH (Ia1)

correspondant à la formule (la) telle que définie précédemment, dans laquelle m et p sont égaux à 0 et R₂ et R₃ représentent un atome d'hydrogène,
ou bien un composé de formule (la₂) :

R₁-(C=O)-O-CH₂-CH(OH)-CH₂-O-C(-O)-R₃ (Ia2)

correspondant à la formule (la) telle que définie précédemment dans laquelle p est égal à 1, m est égal à 0 et R₂ représente un atome d'hydrogène,
ou bien un composé de formule (la₃):

R₁-(C=O)-O-CH₂-CH[O-C(=O)-R₂]-CH₂-O-C(=O)-R₃ (Ia3)

correspondant à la formule (la) telle que définie précédemment dans laquelle m et p sont égaux à 1,
ou bien un mélange de composés de formules (la₁), (la₂) et/ou (la₃).

Comme exemples composés de formules (la₁), (la₂) ou (la₃), il y a les triglycérides d'acides gras ou de mélanges d'acides gras tels que le mélange de triglycérides d'acides gras comportant de 6 à 10 atomes de carbone, commercialisé sous le nom SOFTENOL^{TM} 3819, le mélange de triglycérides d'acides gras comportant de 8 à 10 atomes de carbone, commercialisé sous le nom SOFTENOL^{TM} 3108, le mélange de triglycérides d'acides gras comportant de 8 à 18 atomes de carbone, commercialisé sous le nom SOFTENOL^{TM} 3178, le mélange de triglycérides d'acides gras comportant de 12 à 18 atomes de carbone, commercialisé sous le nom SOFTENOL^{TM} 3100, le mélange de triglycérides d'acides gras comportant 7 atomes de carbone commercialisé sous le nom SOFTENOL^{TM} 3107, le mélange de triglycérides d'acides gras comportant 14 atomes de carbone commercialisé sous le nom SOFTENOL^{TM} 3114 ou le mélange de triglycérides d'acides gras comportant 18 atomes de carbone commercialisé sous le nom SOFTENOL^{TM} 3118, le dilaurate de glycérol, le dioléate de glycérol, l'isostéarate de glycérol, le distéarate de glycérol, le monolaurate de glycérol, le monooléate de glycérol, le monoisostéarate de glycérol, le monostéarate de glycérol ou un mélange de ces composés.

Comme autres exemples de composés de formule (I) telle que définie ci-dessus, il y a les composés de formule (Ib) :

R₁-(C=O)-O-R₃ (Ib)

correspondant à la formule (I) telle que définie précédemment dans laquelle q est égal à 0, ou un mélange de composés de formules (Ib).

Comme exemples de composés de formule (Ib), il y a le myristate d'isopropyle, le myristate d'octyldodécyle, le palmitate d'octyle, le palmitate d'isopropyle ou l'isononate d'isononyle.

Par quantité efficace, on entend plus particulièrement dans le cadre de la présente invention une quantité comprise entre 0.5 % et 85 % en poids de la composition finale et tout particulièrement entre 0.5 % et 45 % en poids de composition finale.

L'invention a plus particulièrement pour objet une composition telle que définie précédemment, caractérisée en ce qu'il s'agit de poudres pressées.

Selon un autre aspect particulier de la présente invention, celle-ci a pour objet une composition telle que définie précédemment, dans laquelle le polymère est un copolymère du méthacrylate de méthyle avec l'un ou l'autre des monomères choisis parmi l'acrylate de butyle, l'acrylate de (1-méthyl propyle), l'acrylate de (2-méthyl propyle) ou l'acrylate de (1,1-diméthyl éthyle), et tout particulièrement une composition telle que définie précédemment sous forme de poudres pressées dans laquelle le polymère utilisé est un copolymère de méthacrylate de méthyle et d'acrylate de butyle.

La poudre compacte obtenue est souple mais résistante, les compacts obtenus sont très significativement moins durs, ce qui facilite le prélèvement de la poudre sur l'applicateur et son relargage sur la peau. Les composés de l'invention améliorent très significativement la compressibilité des poudres pressées.

Les composés selon l'invention peuvent être utilisés dans tous types de poudres pressées quelle qu'en soit l'application finale et sont compatibles avec tous les ingrédients cosmétiques connus de l'homme de métier.

Cette facilité de compression s'accompagne d'un effet sur les propriétés rhéologiques et sensorielles de la poudre formulée, supérieur aux effets obtenus avec les charges dites incompactables, à savoir :
l'amélioration du toucher - augmentation de la douceur de la poudre à l'étalement sur la peau,
l'amélioration de l'uniformité du film de poudre sur la peau,
une meilleure répartition de la couleur, et
l'amélioration de l'adhérence de la poudre sur la peau.

La composition objet de la présente invention est préparée par mélange des poudres de produits de départ puis compression du mélange obtenu. Un avantage supplémentaire de l'utilisation des polymères sus-mentionnés dans la composition objet de la présente invention, réside en ce que le procédé ne comprend pas nécessairement une étape d'extrusion avant l'étape de compression.

Selon un autre aspect de la présente invention, celle-ci a pour objet l'utilisation d'une composition telle que définie précédemment, comme poudre destinée à l'embellissement du visage ou du corps : poudres de soin ou de maquillage telles que les poudres unifiantes du teint, les poudres satinées et hydratantes pour le corps, les fards à joues ou les ombres à paupières ....

Les exemples suivants illustrent l'invention, sans toutefois la limiter.

### Mise en évidence des avantages des compositions selon l'invention par rapport à celles de l'état de la technique.

**Essai (I):** On a préparé des formulations simplifiées comportant 15 % en poids d'un liant hydrophobe, le Schercemol^{TM} 1688, 0,6 % en poids d'un colorant (Ariabel rose) et 84,4 % d'un polymère en poudre de la façon suivante :

La poudre est broyée dans un broyeur à couteaux pendant quelques secondes, puis le liant hydrophobe est ajouté et le mélange est de nouveau broyé pendant quelques secondes. La poudre est ensuite compactée dans une coupelle métallique au moyen d'une compacteuse manuelle KEMWALL^{TM} sous une pression de 30.10⁵ Pa.

L'aspect visuel du compact obtenu est apprécié sous une luminosité standard. Différents critères sont notés :
le niveau de remplissage de la coupelle,
la planéité de la surface,
la friabilité du compact,
la répartition du colorant.

La facilité de prélèvement de la poudre au pinceau est noté sur une échelle de notes allant de 0 à 5, 0 correspondant à un prélèvement nul, 5 correspondant à un prélèvement très facile et une quantité importante de poudre retenue sur le pinceau.

La dureté des compacts est mesurée au moyen d'un texturomètre muni d'une sonde de mesure hémisphérique. Plus la force de compression nécessaire à l'enfoncement de la sonde dans le compact est élevée, plus le compact a une dureté forte.

Les poudres de polymères utilisées conduisant à une formulation de l'état de la technique, sont le MICROPEARL^{TM} M 305 qui un Poly(méthacrylate de méthyle) (formulation A) et le NYLON^{TM}-12 (formulation B).

La poudre de polymère utilisée conduisant à deux formulations selon l'invention, formulation 1 et formulation 2, est une poudre de copolymère de méthacrylate de méthyle et d'acrylate de butyle.

Les résultats des observations sont consignés dans le tableau suivant :

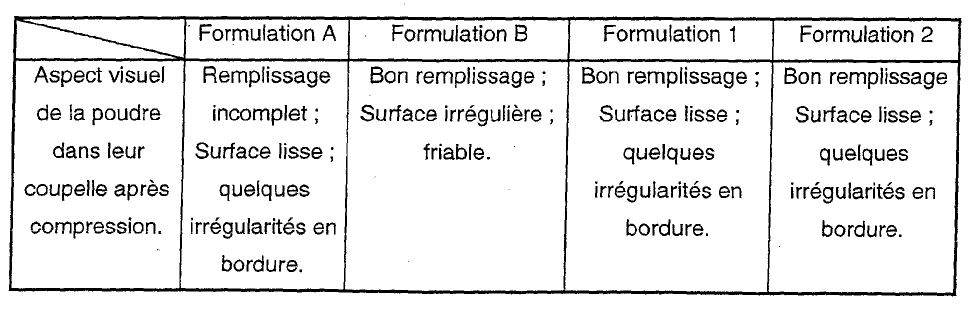

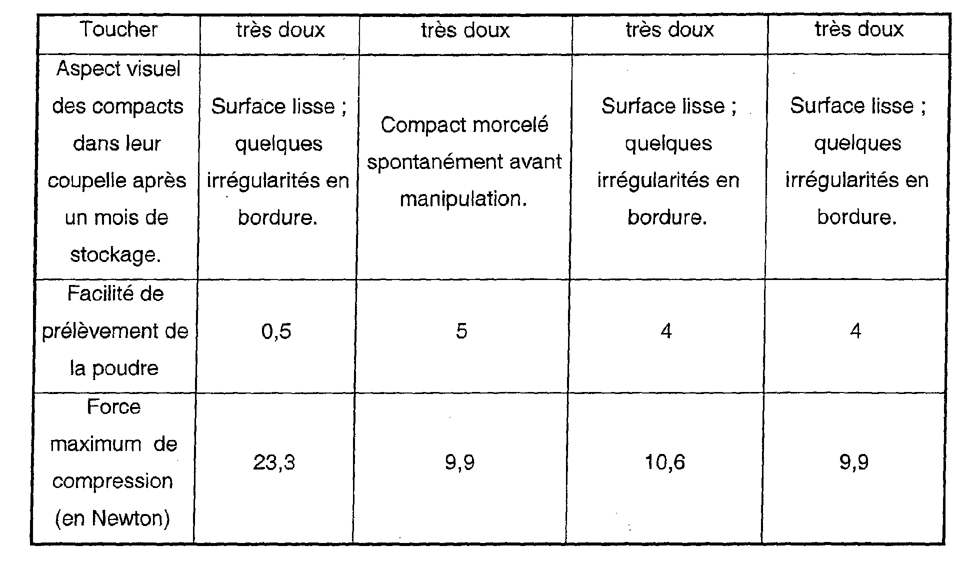

Ces résultats mettent en évidence que les formulations selon l'état de la technique donnent soit des poudres compressées avec une surface lisse mais d'une grande dureté, quasiment impossible à prélever, soit des poudres faciles à prélever mais très fragiles et qui se cassent spontanément au cours du temps. Au contraire, les compositions selon l'invention ont un comportement très différent qui concilie à la fois, la souplesse du compact et le parfait maintien de son intégrité au cours du temps, cela sans altérer les qualités sensorielles de la poudre, la poudre pressée obtenue ayant une bonne cohésion et restant facile à prélever au pinceau.

**Essai (II) :** On a préparé des formulations simplifiées comportant 50 % en poids de Mica 1000, 5 % en poids de LANOL^{TM} 1688, 5 % en poids de DC^{TM} 200, 0,4 % en poids de colorant (dénommé FDC Yellow n°6 Aluminium lake), 0,2 % en poids d'un colorant (Ariabel Sienna), 5 % en poids de polymère en poudre et le complément à 100 % en poids de talc Luzenac^{TM} 00. de la façon suivante :

Les composés pulvérulents sont mélangés puis broyés avec un mélangeur à couteaux. Les liants hydrophobes sont ensuite ajoutés l'un après l'autre avec un broyage du mélange entre chaque ajout. Le mélange final est encore broyé pendant quelques secondes. La poudre est ensuite compactée dans une coupelle métallique au moyen d'une compacteuse manuelle KEMWALL^{TM} sous une pression de 30. 10⁵ Pa. L'aspect visuel des compacts est observé sous une luminosité standard.

La facilité de prélèvement de la poudre au pinceau est notée sur une échelle de 0 à 5 : 0 correspondant à un prélèvement nul, 5 à un prélèvement très facile et une quantité importante de poudre retenue sur le pinceau.

La dureté des compacts est mesurée au moyen d'un texturomètre muni d'une sonde de mesure hémisphérique. Plus la force de compression nécessaire à l'enfoncement de la sonde dans le compact est élevée, plus le compact a une dureté élevée.

Les qualités sensorielles de la formulation sont appréciées par un panel de 15 individus qui notent sur une échelle de 0 à 5 les critères suivants :
La facilité de prélèvement au doigt :
   0 = prélèvement nul ; 5 = prélèvement très facile.
La facilité d'étalement sur la peau :
   0 = étalement difficile, accroche ; 5 = étalement très facile.
Le relargage du colorant :
   0 = mauvaise couverture de la peau, 5 = couleur uniforme.
L'homogénéité du film :
   0 = film hétérogène ; 5 = film parfaitement homogène.
Répartition de la couleur :
   0 = colorant mal réparti ; 5 = colorant uniformément réparti.
Le toucher sur la peau :
   0 = toucher rêche ; 5 = toucher très doux.

La résistance des compacts est évaluée après un test de chute d'une hauteur de 20 cm sur un support en plexiglas. Le test est réalisé sur 10 coupelles, le résultat est exprimé en nombre de chutes moyen provoquant la rupture de la poudre pressée.

Les poudres de polymères utilisées conduisant à une formulation de l'état de la technique sont le MICROPEARL^{TM} M 305 (formulation C) et le NYLON^{TM}-12 (formulation D).

La poudre de polymère utilisée conduisant aux formulations selon l'invention, formulation 3 et formulation 4, est la poudre de copolymère de méthacrylate de méthyle et d'acrylate de butyle.

Les résultats des observations sont consignés dans le tableau suivant :

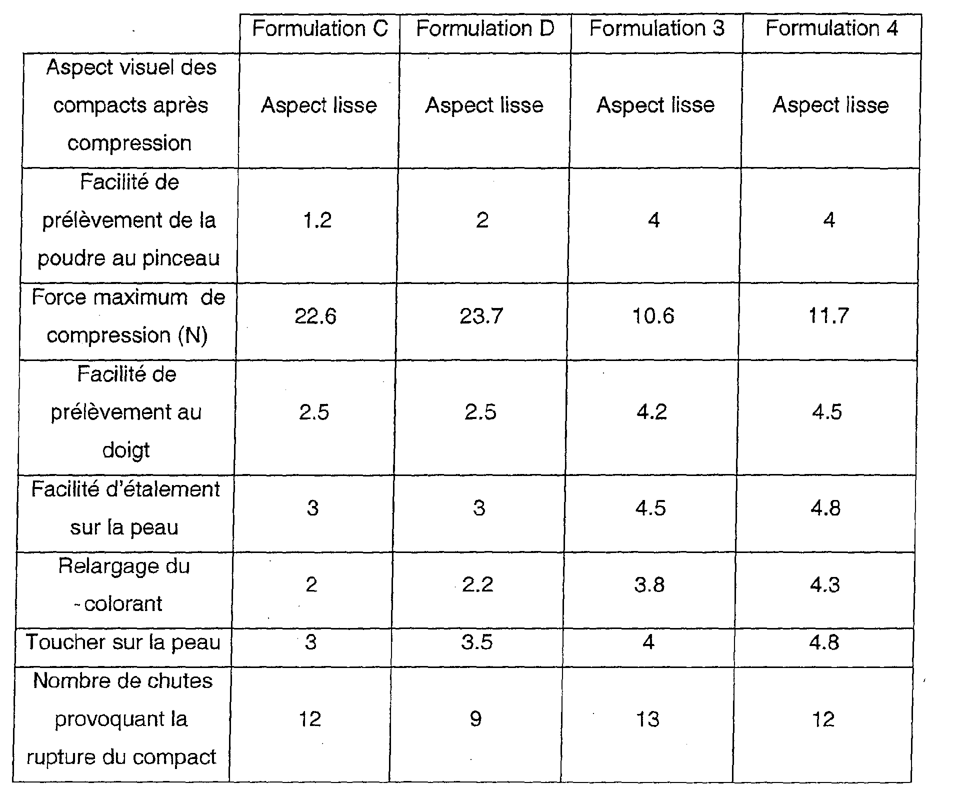

Ces résultats mettent en évidence les avantages obtenus avec les compostions selon l'invention. Les copolymères méthyl méthacrylate / butyl acrylate permettent également d'améliorer les qualités sensorielles de la formulation, les volontaires ont noté particulièrement l'amélioration de la répartition de la couleur et la plus grande adhérence sur la peau de la poudre pressée.

Les poudres de copolymères telles que décrites précédemment peuvent également être avantageusement utilisés comme agent de texture dans toute forme de produit de soin ou de maquillage. L'utilisation des composés selon l'invention améliore très significativement la douceur à l'application et offre des propriétés d'adhérence sur la peau supérieure aux poudres habituelles possédant des particules de forme sphériques.

| Fond teint fluide peaux grasses | | |
|---|---|---|
| A | • Aqua/ Eau | 20.00% |
| | • Butylene glycol | 4.00 % |
| | • PEG-400 | 4.00 % |
| | • PECOSIL PS100 | 1.00 % |
| | • Sodium Hydroxyde | qs pH=9 |
| | • Titanium dioxyde 7.00 % | 7.00 % |
| | • Talc | 2.00 % |
| | • Iron oxyde yellow | 0.80 % |
| | • Iron oxyde red | 0.30 % |
| | • Iron oxyde black | 0.05 % |
| | | |
| B | • MONTANOV L | 1.50 % |
| | • LANOL 99 | 10.00% |
| | • Diisostearyl malate | 10.00 % |
| | | |
| C | • Aqua/Water | QSP 100% |
| | • Tetrasodium EDTA | 0.05 % |
| | • Poudre de copolymère de méthacrylate de méthyle et d'acrylate de butyle | 5.00 % |
| | | |
| D | • SIMULGEL NS | 2.00 % |
| | | |
| E | • SEPICONTROL A5 | 4.00 % |
| | | |
| F | • SEPICIDE HB | 0.30 % |
| | • SEPICIDE Cl | 0.20 % |
| | • Parfum/Fragrance | 0.20 % |

### Mode opératoire

- Mélanger les composés liquides de la phase A puis ajuster le pH à 9 environ avant d'ajouter les pigments. Broyer cette phase pigmentaire avec un broyeur à billes (préparer une quantité supérieure à celle théoriquement nécessaire en raison des pertes).
- Fondre la phase B à 80-85°C.
- Chauffer l'eau à 75°C dans la cuve principale puis ajouter en maintenant le chauffage le MICROPEARL M305, l'EDTA et la pâte pigmentaire A. Introduire ensuite la phase grasse (B); puis déclencher ensuite l'émulseur (rotor stator) et couper le chauffage. Ajouter le SEPICONTROL A5 - laisser tourner l'émulseur quelques minutes à 3000 rpm (selon la quantité fabriquée), puis introduire le SIMULGEL NS et remettre l'émulseur quelques minutes. Refroidir progressivement sous agitation modérée et ajouter vers 30°C les constituants de F. Ajuster le pH final si nécessaire.

Dans les forme galéniques impliquant une dispersion de poudres et se présentant au final sous forme solide, les composés selon l'invention contribuent aux opérations de moulage et préviennent les phénomènes de fendillement fréquemment observés dans le temps avec les agents de texture poudre présentant des particules sphériques, en particulier pour des doses d'utilisation élevées.

| Poudre coulée bonne mine | | |
|---|---|---|
| A | • Talc | 10.00% |
| | • Mica | 20.00 % |
| | • Ariabel Rose | 0.80 % |
| | | |
| B | • Cyclomethicone | 20.00 % |
| | • Poudre de copolymère de méthacrylate de méthyle et | 12.00 % |
| | d'acrylate de butyle | |
| | | |
| C | • LANOL 99) | qs 100% |
| | • Propylene glycol | 15.00% |
| | • Ozokérite | 7.00 % |
| | • SEPIFEEL ONE | 5.00% |

### Mode opératoire

- Broyer la phase A avec un broyeur à couteaux.
- Mélanger B, ajouter B dans A sous agitation - réchauffer le mélange à 50°
- Ajouter C fondu sous agitation
- Couler le mélange et refroidir.

| Rouge à lèvres | | |
|---|---|---|
| A | • Disopropyl dimerdilinoleate | 22,50 % |
| | • Titanium dioxyde | 6,44 % |
| | • Iron oxyde Yellow | 3,04 % |
| | • Iron oxyde Black | 0,36 % |
| | • DC RED 7 | 0,78 % |
| | • DC RED 27 | 0,00 % |
| | • FDC YELLOW 6 | 0,70 % |
| | • FDC Blue 1 | 0,17% |
| | | |
| B | • Disopropyl dimerdilinoleate | 10.70 % |
| | • Ozokérite | 11,75 % |
| | • Pentaérythrityl tetralaurate | 10,00 % |
| | • Octyldocecanol | 8,12 % |
| | • Cire d'abeille | 6,20 % |
| | • Triisotearyl trilinoleate | 5,00 % |
| | • Cetyl palmitate | 4,50 % |
| | • Cire de Carnauba | 2,28 % |
| | • Poudre de copolymère de méthacrylate de méthyle et | 5.00 % |
| | d'acrylate de butyle | |
| | | |
| C | • Sepilift DPHP | 1.00 % |
| | • Parfum | 1.25 % |
| | • Tocopheryl acetate | 0.20 % |

### Mode opératoire :

- Broyage de la pâte pigmentaire au dispermat
- Chauffage de la phase A et B au bain marie à 90°C
- Introduction progressive des cires au fur et à mesure de la fonte
- Mélange des deux phases fondues 10 ' à l'ancre
- Introduction des ingrédients de C + mélange 3' à l'ancre
- Coulage de la pâte dans les moules

Les caractéristiques des produits utilisés dans les exemples précédents sont les suivantes :
Le SEPICIDE^{TM} Cl, imidazoline urée, est un agent conservateur commercialisé par la société SEPPIC.
Le SEPICIDE^{TM} HB, qui est un mélange de phénoxyéthanol, de méthylparaben, d'éthylparaben, de propylparaben et de butylparaben, est un agent conservateur commercialisé par la société SEPPIC.
Le PECOSIL^{TM} PS100 est du diméthicone copolyol phosphate commercialisé par la société PHOENIX.
Le LANOL^{TM} 99 est de l'isononyl isononanoate commercialisé par la société SEPPIC.
Le SEPIFEEL^{TM} ONE est un mélange de palmitoylproline, de palmitoyl glutamate de magnésium et de palmitoyl sarcosinate de magnésium, tel que ceux décrits dans FR 2787323.
Le SEPICONTROL^{TM} A5 est un mélange capryloyl glycine, sarcosine, extrait de cinnamon zylanicum, commercialisé par la société SEPPIC,
Le SIMULGEL^{TM} NS est un latex inverse épaississant tel que décrit dans la demande internationale WO 99/36445.

## Revendications

1. Utilisation d'un polymère ou d'un mélange de polymères choisis parmi les copolymères ou les terpolymères du méthacrylate de méthyle avec un ou plusieurs de monomères choisis parmi l'acrylate de butyle, l'acrylate de (1-méthyl propyle), l'acrylate de (2-méthyl propyle), l'acrylate de (1,1-diméthyl éthyle), le méthacrylate de butyle, le méthacrylate de (1-méthyl propyle), le méthacrylate de (2-méthyl propyle) ou le méthacrylate de (1,1-diméthyl éthyle), comme agent de texture dans des compositions cosmétiques ou pharmaceutiques formulées sous forme de poudres, applicables sur le corps humain ou animal.

2. Utilisation telle que définie à la revendication 1, dans laquelle les compositions cosmétiques ou pharmaceutiques sont des poudres pressées.

3. Utilisation telle que définie à l'une des revendications 1 ou 2, d'un copolymère du méthacrylate de méthyle avec l'un ou l'autre des monomères choisis parmi l'acrylate de butyle, l'acrylate de (1-méthyl propyle), l'acrylate de (2-méthyl propyle) ou l'acrylate de (1,1 - diméthyl éthyle).

4. Utilisation telle que définie à la revendication 3, d'un copolymère de méthacrylate de méthyle et d'acrylate de butyle.

5. Composition cosmétique ou pharmaceutique formulée sous forme d'une poudre, applicable sur le corps humain ou animal, **caractérisé en ce qu'**elle comprend :
- une quantité efficace d'un polymère ou d'un mélange de polymères tel que défini à l'une des revendications 1 à 4 et,
- au moins un composé cosmétiquement acceptable.

6. Composition telle que définie à la revendication 5, **caractérisée en ce qu'**il s'agit de poudres pressées.

7. Composition telle que définie à l'une des revendications 5 ou 6, dans laquelle le polymère est un copolymère du méthacrylate de méthyle avec l'un ou l'autre des monomères choisis parmi l'acrylate de butyle, l'acrylate de (1-méthyl propyle), l'acrylate de (2-méthyl propyle) ou l'acrylate de (1,1 - diméthyl éthyle).

8. Composition telle que définie à la revendication 6, dans laquelle le polymère utilisé est un copolymère de méthacrylate de méthyle et d'acrylate de butyle.

9. Utilisation d'une composition telle que définie à l'une des revendications 4 à 8, comme poudre destinée à l'embellissement du visage ou du corps : poudres de soin ou de maquillage telles que les poudres unifiantes du teint, les poudres satinées et hydratantes pour le corps, les fards à joues ou les ombres à paupières.
